# EUROPEAN PATENT APPLICATION

(11) **EP 4 209 212 A1**
(43) Date of publication of application: **12.07.2023**
(21) Application number: 22150365.9
(22) Date of filing: 05.01.2022
(51) Int. Cl.: A61K 31/365, A61P 11/00, A61P 11/02, A61K 9/00, A61K 9/113, A61K 31/7048, A61P 31/14, A61P 31/18, A61M 3/00, A61M 5/00, A61M 11/00, A61K 9/14

(54) **AQUEOUS COMPOSITION COMPRISING AVERMECTINS**

(71) Applicant: HWI pharma services GmbH, 76761 Rülzheim (DE)
(72) Inventor: Wissel, Stefan, 76829 Landau (DE); Rischer, Matthias, 60388 Frankfurt (DE); Wissel, Philipp, 75180 Pforzheim (DE); Häberlein, Felix, 76829 Landau (DE)
(74) Representative: Patentanwälte Ruff, Wilhelm, Beier, Dauster & Partner mbB

(57) **Abstract**

An aqueous composition is provided which comprises at least one water-soluble or water-dispersible natural or synthetic polymer in an amount from 0.1% by weight to 10% by weight, based on the total weight of the composition, at least one first surfactant in an amount from 0.1% by weight to 7.5% by weight, based on the total weight of the composition, at least one second surfactant in an amount from 0.1% by weight 7,5% by weight, based on the total weight of the composition, at least one active pharmaceutical ingredient selected from the group of avermectins in an amount from 2% by weight to 15% by weight, based on the total weight of the composition, and water in an amount from 60% by weight to 97.7% by weight.

All weight indications mentioned above are based on the total weight of the aqueous composition.

Preferably, the at least one active pharmaceutical ingredient selected from the group of avermectins is ivermectin.

Further, the aqueous composition is preferably for use in a pharmaceutical composition or medicament, wherein preferably said use is for the prevention or treatment of viral diseases, in particular for use in the prevention or treatment of a SARS-Covid-2 infection or of a HIV infection.

## Description

### TECHNICAL FIELD AND PRIOR ART

The present invention relates to a novel aqueous composition comprising avermectins in particular for nasal applications. The present invention also relates to a discharging device comprising this aqueous composition and to the use of this aqueous composition in a pharmaceutical composition or medicament. Further this system of aqueous composition and device is particular useful in the treatment of viral diseases via a nasal application.

Because of their anti-parasitic properties avermectins are often used as medication to treat orally parasitic worm infections in adults and children. The use of avermectins for this purpose has been extensively studied and is considered as a safe medication. However, avermectins have a low solubility in aqueous systems and are sensitive to oxidation which may lead to impurities and by-products. To prevent oxidation oral dosage forms of avermectin often comprise strong oxidation inhibitors like Butylhydroxytoluol (Driponin^{®} 3mg) or Butylhydroxyanisol (Scabioral^{®}). However, these oxidation inhibitors show a low solubility in aqueous systems as well.

In addition to the low solubility and high oxidation sensitivity, avermectins possess a low permeability through organic tissue which impairs the release profile and bioavailability of avermectins. To improve the bioavailability of avermectins the application of a highly concentrated avermectin medication can be a solution. However, a high concentration of avermectins in an aqueous composition is nearly impossible to facilitate.

Therefore, an aqueous composition of avermectins cannot facilitate an acceptable solubility of avermectins and oxidation inhibitors in an aqueous composition for medical use and a suitable concentration of avermectins which is needed to assure a good bioavailability.

It is an object of the present invention to provide a novel aqueous composition comprising avermectins, which has superior properties with regard to solubility, stability and bioavailability of avermectins, compared to conventional compositions comprising avermectins.

### SUMMARY OF THE INVENTION

The above object and further objects are solved by an aqueous composition comprising avermectins according to claim 1, a discharging device according to claim 16 and an aqueous composition for use in a pharmaceutical composition or medicament according to claim 17 and claim 18. Preferred embodiments are defined in the dependent claims. Preferably, but not necessarily, said aqueous composition is especially suitable for a nasal administration. The wording of all claims is explicitly incorporated into the content of this description by reference.

The present invention provides an aqueous composition comprising
- at least one water-soluble or water-dispersible natural or synthetic polymer in an amount from 0.1% by weight to 10% by weight, based on the total weight of the composition,
- at least one first surfactant in an amount from 0.1% by weight to 7.5% by weight, based on the total weight of the composition,
- at least one second surfactant in an amount from 0.1% by weight to 7.5% by weight, based on the total weight of the composition,
- at least one active pharmaceutical ingredient selected from the group of avermectins in an amount from 2% by weight to 15% by weight, based on the total weight of the composition, and
- water in an amount from 60% by weight to 97.7% by weight, based on the total weight of the composition.

In a preferred embodiment of the invention the inventive aqueous composition is sterile.

The terms used in the claims and in the overall description are defined as follows.

The term "aqueous composition" as used herein, means a composition which is free of organic solvents or non-aqueous components.

The term "free of organic solvents or non-aqueous components" as used herein means that less than 5% by weight, in particular less than 2% by weight, preferably less than 1% by weight organic solvents or non-aqueous components are present in the aqueous composition, based on the total weight of the composition.

The term "polymer" as used herein, means a substrate, preferably a hydrophilic (water-soluble) substrate, used in the process of pharmaceutically active compound delivery, which serves to regulate the viscosity and to improve the stability of the included active pharmaceutical ingredient in the aqueous composition, in particular the stability of the included multiparticulates of said active pharmaceutical ingredient in the aqueous composition.

The term "surfactant" as used herein, means a compound, preferably a hydrophilic (water-soluble) compound, that lowers the surface tension between two liquids or between a liquid and a solid in a composition, enabling or supporting the stabilization of the included active pharmaceutical ingredient, in particular the stability of the included multiparticulates of said active pharmaceutical ingredient in the aqueous composition.

The term "active pharmaceutical ingredient" (API) as used herein, means any substance or combination of substances used in a pharmaceutical composition or in a medicament or in a drug, intended to furnish the desired pharmacological activity, preferably avermectins, more preferably ivermectin. In other words, the "active pharmaceutical ingredient" is the component in the pharmaceutical composition or medicament or drug that is biologically active. Other terms and expressions with the same meaning are "pharmaceutically active compound", "pharmaceutically active agent" or "pharmaceutically active ingredient".

The term "multiparticulate" as used herein, means a plurality of particles of at least one active pharmaceutical ingredient, wherein the particles are of the same size or of different size.

The term "sterile" as used herein, means free from living germs or microorganisms, in particular aseptic.

The term "mucoadhesion" as used herein, means an adhesion at organic tissues which are mostly covered by mucus or mucous membranes such as nose and mouth.

A syringe is a reciprocating pump (including piston pump, plunger pump and diaphragm pump) consisting of a plunger/piston tightly fitting within a cylindrical tube. This construction allows the syringe to take in and to expel liquid (or gas) through a discharge orifice at one end of the tube.

A spray device is able to generate an aerosol, namely a suspension of fine liquid droplets in air or another gas, by a spray system with the means of atomization. Such spray devices are e.g. used in nasal sprays which deliver medication locally in the nasal cavities or systemically. Blow-fill-seal (BFS) ampoules are formed, filled, and sealed in a continuous process, even under sterile conditions. These ampoules are widely used within the pharmaceutical industry and are made often from polyethylene or polypropylene. An important field of application is the production of eye drops.

In preferred embodiments of the invention the aqueous composition can additionally comprise additives which can further optimize the properties of the inventive composition, e.g. its physical or chemical properties. Preferably these additives are present in the inventive composition in a total amount of less than 10 % by weight, in particular in a total amount of less than 5 % by weight. In this context it is even further preferred, if the total amount of additives in the inventive aqueous composition does not exceed 3 % by weight.

The additives can be taste masking excipients, preservatives, antioxidants, chelating agents, and other additives which can be present in an aqueous composition for pharmaceutical use.

Taste masking additives are substances or chemicals which are added to improve the taste of desired compositions. According to the invention sugars or sugar alcohols can be used as taste masking agents.

Preservatives are substances or chemicals which are added to (among others) pharmaceutical compositions to prevent microbial growth or other undesired changes of the composition. According to the invention parabens can be used as preservatives. Other examples for useful preservatives are sorbic acid, sodium sorbate and other sorbates, as well as benzoic acid and benzoates.

Antioxidants are compounds that inhibit oxidation, i.e. a chemical reaction which can produce free radicals. Said free radicals can lead to chain reactions which may damage the cells of an organism.

Antioxidants which can be used according to the invention are naturally occurring substances, like citric acid and ascorbic acid (vitamin C). A synthetic antioxidant is e.g. butylated hydroxytoluene (BHT).

Chelating agents are chemical compounds which form complexes with metal ions. They are used e.g. as additives to bind metal ions, in particular heavy-metal ions in the form of a complex (chelate). The chelating agents useful in binding metal ions, especially heavy-metal ions are known to a person skilled in the art. An important example for such a chelating agent is ethylenediaminetetraacetic acid (EDTA).

According to the invention the at least one polymer is preferably present in the inventive aqueous composition in an amount from 1% by weight to 10% by weight, preferably from 1% by weight to 5% by weight, more preferably in an amount from 2% by weight to 3% by weight, based on the total weight of the aqueous composition.

The above mentioned amounts of the at least one polymer have been found to be particularly advantageous, because the inventive aqueous composition comprising the at least one polymer in amounts above 5 % by weight, based on the total weight of the aqueous composition, no longer supports some of the advantages of the inventive aqueous composition and amounts above 10 % by weight, based on the total weight of the aqueous composition, no longer supports most of the advantages of the inventive aqueous composition since the aqueous composition can become too viscous and unmanageable in nasal spray compositions, in particular when using hydroxypropylcellulose (HPC) or hydroxypropylmethylcellulose (HPMC). However, polymers like polyvinylpyrrolidon polymer (PVP) in amounts above 5 % by weight as well as above 10% by weight, based on the total weight of the aqueous composition, can be used without the inventive aqueous composition being too viscous and unmanageable in nasal spray compositions. However, PVP shows a lower mucoadhesive effect than hydroxypropylcellulose (HPC) or hydroxypropylmethylcellulose (HPMC).

As defined above the at least one polymer present in the inventive aqueous composition is a water-soluble or water-dispersible natural or synthetic polymer, which can form viscous solutions in aqueous systems and in addition, enable the adhesion of the inventive aqueous compositions to tissues as nose or buccal areas in the mouth (mucoadhesion), which is difficult due to the coverage of these tissues by a mucus layer or mucous membranes which make adhesion challenging. In principle said polymers can be selected from collagen derivatives, polyalkylene glycols, in particular polyethyleneglycols, polyglycerols, alginates, carrageenan, pectins, tragacanth gum, gums, especially gum arabic, and/or dextran.

A balance between viscosity and adhesion of such aqueous composition and spray device or blow-fill-seal ampoule application must therefore be carefully considered when choosing the polymer and the amount of the polymer in the inventive aqueous composition.

According to the invention the at least one polymer preferably comprises at least one cellulose derivative, in particular hydroxypropylmethylcellulose, polyvinylpyrrolidones and derivatives thereof, polyoxyethylene-polyoxypropylene copolymers, tocopherol-polyethylene glycol, and polyalkylene glycols, in particular polyethylene glycols. All compounds are toxicological proven and can e.g. be used for topical nasal applications. Besides its influence on viscosity and adhesion properties of the inventive aqueous composition the polymer is responsible to stabilize the active pharmaceutical ingredient in the aqueous composition, in particular the particles, especially micron or submicron particles of said active pharmaceutical ingredient.

It was found that the inventive aqueous composition is (because of the chosen polymers) especially useful for providing a good mucoadhesion of the inventive aqueous composition which allows a prolonged bioavailability of a therapeutically effective amount of active pharmaceutical ingredients after administration, in particular after nasal administration.

In a preferred embodiment of the invention the at least one polymer is preferably selected from a group comprising of the cellulose derivative hydroxypropylcellulose (HPC) or hydroxypropylmethylcellulose (HPMC). HPC polymers, Grade SL, and HPMC polymers, Grade 603, are further preferred.

Hydroxypropylmethylcellulose (HPMC) has been found to regulate the viscosity and mucoadhesion in the inventive aqueous systems especially advantageous.

In another preferred embodiment of the invention the at least one polymer is preferably selected from a group comprising grades of a polyvinylpyrrolidon polymer (PVP), namely PVP grades PF17, K25 and K30.

In another preferred embodiment of the invention the at least one polymer is preferably selected from a group comprising a polyvinylpyrrolidon copolymer with vinylacetate (VA), in particular the copolymer PVP VA 64.

In another preferred embodiment of the invention the at least one polymer is selected from a group of polyoxyethylene-polyoxypropylene copolymers called Poloxamers. Poloxamers are nonionic triblockk copolymess of a hydrophobic chain of polyoxypropylene flanked by two hydrophilic chains of polyoxyethylene. Preferably the at least one polymer is a Poloxamer type 188 or type 407 copolymer.

In a more preferred embodiment of the invention the at least one polymer is selected from a group comprising polyoxyethylene-polyoxypropylene copolymers under the trademark Kolliphor^{®} (BASF). The most preferred polymers are Kolliphor P188 and Kolliphor P407.

According to the invention the at least one polymer preferably comprises at least one tocopherol-polyethylene glycole 1000 succinate derivative or mixtures thereof.

In another preferred embodiment of the invention the at least one polymer preferably comprises the D-alpha-tocopherol-polyethylene glycole 1000 succinate derivative or mixtures thereof.

In another preferred embodiment of the invention the at least one polymer preferably comprises the D-alpha-tocopherol-polyethylene glycole 1000 succinate type.

In another preferred embodiment of the invention the at least one polymer is preferably selected from a group comprising Carboxyvinylpolymers, wherein homopolymers are further preferred. A suitable group of said polymers is sold under the trademark Carbopol^{®} (Lubrizol), e.g. the preferred homopolymer Carbopol 971.

In a preferred embodiment of the invention the viscosity of the inventive aqueous composition at a temperature of approx. 20 °C - 35 °C is between 1 mPas and 1000 mPas, preferably between 1 mPas and 100 mPas, more preferably between 1 mPas and 50 mPas.

According to the invention viscosity normally is measured with a viscometer, in particular a rheometer. A rheometer usually is a laboratory device measuring flow characteristics of a liquid, suspension or slurry in response to applied forces. A common device type is a so-called shear rheometer, in which a shear stress is applied to the corresponding material. Devices which can be used to measure the viscosity of the non-aqueous compositions according to the invention are rheometers from Anton Paar Germany GmbH, Ostfildern, Germany (e.g. MCR 101).

According to the invention the at least one first surfactant is preferably present in the inventive composition in an amount from 0.1% by weight to 5% by weight, preferably from 0.1% by weight to 1% by weight, more preferably in an amount from 0.1% by weight to 0.5% by weight, based on the total weight of the composition.

The above mentioned amounts of the at least one first surfactant have been found to be particularly advantageous, because the inventive aqueous composition comprising the at least one first surfactant in amounts above 5 %, based on the total weight of the aqueous composition, no longer supports some of the advantages of the inventive aqueous composition and amounts above 7.5 % by weight, based on the total weight of the aqueous composition, no longer supports most of the advantages of the inventive aqueous composition since the aqueous composition can show a foam development, in particular when using polyoxyethylen(20)-sorbitan-monooleat (Tween^{®} 80).

According to the invention the at least one first surfactant preferably comprises at least one ionic or non-ionic first surfactant. The first surfactants described below are especially useful for improving the wettability and stabilization of the active pharmaceutical ingredient in the aqueous composition to ensure a homogeneous distribution of active pharmaceutical ingredients, preferably hydrophobic active pharmaceutical ingredient particles, in the inventive aqueous composition.

It was found that the inventive aqueous composition is (because of the chosen first surfactants) especially useful for avoiding an oxidation of the active pharmaceutical ingredient, which may lead to impurities and by-products, and increasing the stability of the active pharmaceutical ingredient. Therefore, the use of antioxidants to prevent oxidation of active pharmaceutical ingredients, in particular from the group of avermectins, can be avoided or the amount of antioxidants in the aqueous composition can be reduced.

In a preferred embodiment of the invention the at least one ionic first surfactant is selected from a group comprising dioctyl sulfosuccinate ester or sodium dodecyl sulphate or mixtures thereof.

In another preferred embodiment of the invention the at least one non-ionic first surfactant is selected from a group comprising polysorbate esters, e.g. polyoxyethylen(20)-sorbitan-monolaurat (Tween^{®} 20), polyoxyethylen(20)-sorbitan-monopalmitat (Tween^{®} 40), polyoxyethylen(20)-sorbitan-monostearat (Tween^{®} 60), polyoxyethylen(20)-sorbitan-tristearat (Tween^{®} 65) and polyoxyethylen(20)-sorbitan-monooleat (Tween^{®} 80). Preferred compounds are sold under the trademark Tween^{®} (Croda).

In a more preferred embodiment of the invention the at least one non-ionic first surfactant is polyoxyethylen(20)-sorbitan-monooleat (Tween^{®} 80).

According to the invention the at least one second surfactant is preferably present in the composition in an amount from 0.1% by weight to 5% by weight, preferably from 0.1% by weight to 2.5% by weight, more preferably in an amount from 1% by weight to 2% by weight, based on the total weight of the composition.

The above mentioned amounts of the at least one second surfactant have been found to be particularly advantageous, because the inventive aqueous composition comprising the at least one second surfactant in amounts above 5 %, based on the total weight of the aqueous composition, no longer supports some of the advantages of the inventive aqueous composition and amounts above 7.5 % by weight, based on the total weight of the aqueous composition, no longer supports most of the advantages of the inventive aqueous composition since the aqueous composition can lead to fluidization of membranes and thus can lead to biological incompatibilities, in particular when using sodium glycocholate (SGC).

According to the invention the at least one second surfactant preferably comprises at least one ionic or non-ionic second surfactant. The second surfactants described below are especially useful for improving the wettability and stabilization of the active pharmaceutical ingredient in the aqueous composition to ensure a homogeneous distribution of active pharmaceutical ingredients, preferably hydrophobic active pharmaceutical ingredient particles, in the inventive aqueous composition.

It was found that the inventive aqueous composition is (because of the chosen second surfactants) especially useful for avoiding an oxidation of the pharmaceutically active ingredient from the group of avermectins, which may lead to impurities and by-products, and for increasing the stability of the pharmaceutically active ingredient from the group of avermectins. Therefore, the use of antioxidants to prevent oxidation of active pharmaceutical ingredients, in particular from the group of avermectins, can be avoided or the amount of antioxidants in the aqueous composition can be reduced.

Further, it was found that the at least one second surfactants described below are especially useful to obtain an increased biological activity (resorption) by improving the release profile and the bioavailability of hydrophobic particles, preferably hydrophobic active pharmaceutical ingredient particles, in the inventive aqueous composition and for increasing the stability of hydrophobic particles, preferably hydrophobic active pharmaceutical ingredient particles, in the inventive aqueous composition. The increased local and systemic effect has to be understood as the time needed for the active pharmaceutical ingredient to be fast released from the applied formulation, in particular on the nasal mucosa, into the organic tissue to enable the increased therapeutic effect of the adsorbed ingredients.

In a preferred embodiment of the invention the at least one ionic second surfactant is selected from a group comprising sodium taurocholate (STC), sodium glycocholate (SGC) and sodium deoxycholate (SDC) or mixtures thereof.

In a more preferred embodiment of the invention the at least one ionic second surfactant is sodium glycocholate (SGC).

In another preferred embodiment of the invention the at least one non-ionic second surfactant is selected from a group comprising tocopherol-polyethylene glycols or mixtures thereof.

It was found that the inventive aqueous composition is (because of the chosen combination of polymers and first surfactants and second surfactants) especially useful for providing an in vivo prolonged therapeutic effect of a therapeutically effective amount of the active pharmaceutical ingredients, in particular from the group of avermectins, after administration (bioavailability), preferably at internal organs, in particular after nasal administration, and an advantageous stability of the active pharmaceutical ingredients, in particular from the group of avermectins.

According to the invention, the aqueous composition is preferably a prolonged release composition, which provides an equally, prolonged activity, of the active pharmaceutical ingredient, in particular activity in the organic tissue, in particular of a human body, over a period of at least 1 hour, more preferably of at least 2 hours after a single administration.

According to the invention the at least one active pharmaceutical ingredient selected from the group of avermectins is preferably ivermectin.

Avermectins are a group of 16 macrocyclic lactone derivatives which are mainly known to treat parasitic worms and insect pests. These compounds and their chemical structures are known to a person skilled in the art.

An important representative of the compounds known as avermectins is ivermectin which can be preferably present in the aqueous composition according to the invention.

In a preferred embodiment of the invention the at least one active pharmaceutical ingredient from the group of avermectins has a (low) solubility in water at room temperature (22-26°C) of < 1 mg/ml, preferably < 0,1 mg/ml.

The low solubility of active pharmaceutical ingredients negatively affects the uptake of said ingredients into the body, in particular in the nasal mucosa of the body, after administration. As a consequence, a therapeutic effect may not be achieved by a single administration, but a local depot formulation having a high concentration of active pharmaceutical ingredients using the intrinsic low solubility of the active pharmaceutical ingredients in a suitable aqueous composition to obtain a prolonged activity of the ingredients over the desired time interval. This is achieved by the inventive aqueous composition as described above. The prolonged therapeutic effect has to be understood as the time needed for the active pharmaceutical ingredient to be fast released from the aqueous composition into the organic tissue to enable the prolonged local therapeutic effect of the adsorbed ingredients, especially the corresponding multiparticulates known for e.g. for nanoparticles consisting of PLGA particles loaded with a fluorescence marker in bovine nasal mucosa [Mohammed A. Albarki and Maureen D. Donovan, AAPS PharmSciTech , 06/2020].

According to the invention the at least one active pharmaceutical ingredient selected from the group of avermectins, in particular ivermectin, is preferably present in the composition in an amount from 4 % by weight to 15 % by weight, preferably from 2 % by weight to 10 % by weight, more preferably in an amount from 2 % by weight to 8 % by weight, based on the total weight of the composition.

It was found that the inventive aqueous composition is (because of the chosen combination of polymers and first surfactants and second surfactants) especially useful for providing a high concentration of avermectins in the aqueous composition improving the in vivo prolonged therapeutic effect of a therapeutically effective amount of the active pharmaceutical ingredient from the group of avermectins after administration (bioavailability), preferably at internal organs, in particular after nasal administration.

According to the invention the aqueous composition can further comprise at least one buffer, wherein preferably the at least one buffer is present in an amount from 0.001% by weight to 10% by weight, preferably from 0.1% by weight to 8% by weight, more preferably from 0.14% by weight to 6% by weight, based on the total weight of the composition.

A buffer or buffer system is an aqueous (buffer) solution consisting of a mixture of a weak acid and its conjugate base, or vice versa. The pH value of this solution changes only little when a small amount of a strong acid or base is added.

According to the invention the at least one buffer preferably comprises at least one inorganic salt or at least one organic compound. The buffers described below are especially useful for inhibiting the oxidation of the active pharmaceutical ingredient, in particular from the group of avermectins. Preferably the buffer contributes to an advantageous inhibition of oxidation of the active pharmaceutical ingredient from the group of avermectins in combination with the at least one first surfactant and at least one second surfactant.

In a preferred embodiment of the invention the at least one inorganic salt is selected from a group comprising monosodium phosphate, disodium phosphate, sodium bicarbonate, dicalcium phosphate, tris(hydroxymethyl) aminomethane (TRIS), sodium citrate and sodium acetate.

In a more preferred embodiment of the invention the at least one buffer is sodium citrate.

In a preferred embodiment of the invention the at least one organic compound is selected from a group comprising glycine, gluconic acid and chelating agents, in particular ethylenediaminetetraacetic acid (EDTA).

According to the invention the at least one active pharmaceutical ingredient present in the aqueous composition preferably has a mean particle size of 0.01 to 20 µm, preferably of less than 0.5 µm, in particular of less than 0.5 µm, with a preferred lower limit of 0.1 µm. Thus, the mean particle size of the active pharmaceutical ingredient is in the submicron range, or even in the nano range.

In other preferred embodiments of the invention the active pharmaceutical ingredient has a mean particle size of less than 10 µm, preferably of less than 5 µm, wherein a mean particle size between 1 µm and 4 µm is even further preferred. Thus, the mean particle size of the active pharmaceutical ingredient is in the micron range.

According to the invention the at least one active pharmaceutical ingredient present in the aqueous composition preferably consists of at least two groups of particles with different (mean) particle sizes. Preferably at least one of these groups (fractions) consists of particles with sizes in the micron range (preferred mean size < 3 µm, i.e. < 3000 nm) and the other group consists of particles with sizes in the submicron or nano range (preferred mean size < 0,5 µm, i.e. < 500 nm). In particular the at least one active pharmaceutical ingredient from the group of avermectins is present in two groups of particles, namely in a mean particle size of 0.1 µm to 0.3 µm of one particle fraction (the submicron fraction) and a mean particle size of 1 to 10 microns of another particle fraction (the microparticle fraction).

The term "mean particle size" as used herein, relates to a particle whose dimensions, in particular its diameter, preferably mean diameter, lies in a range from 0,01 to 10 µm, preferably in the ranges as defined above. In this context, the term "diameter" is to be understood in the case of a spherical particle to mean the diameter of the sphere, i.e. twice the radius of the sphere. In the case of a non-spherical particle, the term "diameter" is to be understood as the largest possible distance that two points along a circumference of the particle can take from each other.

The mean particle size distribution is preferably obtained by laser diffraction. In a laser diffraction measurement, a laser beam passes through a dispersed particulate sample and the angular variation in intensity of the scattered light is measured. Large particles scatter light at small angles relative to the laser beam and small particles scatter light at large angles. The angular scattering intensity data is then analyzed to calculate the size of the particles that created the scattering pattern using the Mie theory of light scattering. The particle size is reported as a volume equivalent sphere diameter. The folded optical design in the Mastersizer 3000 (from Malvern Panalytical), which was in particular used for the measurements, provides a particle size range from 10 nm up to 3.5 mm using a single optical measurement path. The Mastersizer 3000 uses a sequential combination of measurements with red and blue light sources to measure across the entire particle size range.

The above described mean particle size of the at least one active pharmaceutical ingredient from the group of avermectins was found to be especially useful to obtain a homogenous distribution of said ingredients in the inventive aqueous composition.

In an embodiment of the invention the desired particle size of the at least one active pharmaceutical ingredient has been preferably obtained by grinding, ball milling, wet bead milling, high pressure homogenization, homogenization, micronisation or a combination of at least two of the described methods.

In a further embodiment of the invention the at least one active pharmaceutical ingredient has been preferable stored in an inert container to avoid degradation after milling or grinding. In particular brown glass bottles closed with a tight seal and cap have been found out to be suitable to avoid significant degradation over storage time.

The low solubility of the active pharmaceutical ingredient selected from the group of avermectins negatively affects the uptake of said ingredients into the body, in particular in the nasal mucosa of the body, after administration. As a consequence, a therapeutic effect may not be achieved by a single administration. It was found that the inventive aqueous composition uses (because of the chosen combination of polymers, first surfactants and second surfactants) the intrinsic low solubility of the active pharmaceutical ingredients from the group of avermectins, preferably in a suitable nasal spray composition, to obtain an increased biological activity (resorption) by improving the release profile and the bioavailability of hydrophobic particles, preferably hydrophobic pharmaceutically active ingredient particles, in the inventive aqueous composition and to increase the stability of hydrophobic particles, preferably hydrophobic active pharmaceutical ingredient particles, in the inventive aqueous composition. Especially the particle size as defined above promote this effect.

Further, due to the above described particle size of the at least one active pharmaceutical ingredient the prolonged therapeutic effect of the inventive composition into a body, after administration at organic tissues, is especially advantageous in comparison to other conventional compositions comprising pharmaceutically active ingredients. Due to the advantageous prolonged therapeutic effect of the inventive aqueous composition, lesser amounts of active pharmaceutical ingredients in the composition are needed to achieve the same therapeutical effect as conventional compositions. This effect is supported by the permeation and release profiles through an artificial membrane in a Franzcell equipment (see figures below). Depending on the use of submicron particles, of micron particles or of a mixture thereof the effect can be triggered to the target profile for the therapeutic effect in-vivo.

The Franz Diffusion Cell is a comparably simple, reproducible testing device for measuring the in vitro release of pharmaceutically active ingredients or drugs from aqueous compositions, gels, creams and ointments. It consists of two chambers which are separated by a membrane. The test product is provided in one chamber, e.g. a top chamber and the amount of the ingredient or drug which has permeated through the membrane into the other chamber, e.g. a bottom chamber is determined over time.

The term "prolonged therapeutic effect of an active pharmaceutical ingredient into a body" as used herein, means an fast uptake of an active pharmaceutical ingredient into a mammal body, in particular a human and/or an animal body, in particular into the nasal cavity or nasal mucosa, followed by the prolonged local therapeutic effect by the mucosal absorbed active ingredients, especially the corresponding multiparticulates of said ingredients.

According to the invention an aqueous composition is preferred, wherein:
- the at least one water-soluble or water-dispersible natural or synthetic polymer is hydroxypropylmethylcellulose, present in an amount from 2% by weight to 3% by weight, based on the total weight of the composition,
- the at least one first surfactant is polyoxyethylen(20)-sorbitan-monooleat (Tween^{®} 80), present in an amount from 0.1% by weight to 0.5% by weight, based on the total weight of the composition,
- the at least one second surfactant is sodium glycocholate (SGC), present in an amount from 1% by weight to 2% by weight, based on the total weight of the composition,
- the at least one active pharmaceutical ingredient selected from the group of avermectins is ivermectin, present in an amount from 2% by weight to 8% by weight, based on the total weight of the composition,
- water is present in an amount from 86% by weight to 94.9% by weight, based on the total weight of the composition, and
- the at least one active pharmaceutical ingredient has a mean particle size of 0.1 µm to 0.5 µm.

The aqueous composition mentioned above was found to be especially advantageous, concerning the adhesion properties and the viscosity properties of the inventive aqueous composition as well as the stability, solubility and uptake of an active pharmaceutical ingredient, especially with a mean particle size of 0.1 µm to 0.5 µm, from the inventive aqueous composition into the body providing a sustained release of the compound for a period of at least 3 days, preferably at least 5 days, after a single administration at organic tissue (bioavailability).

According to the invention an aqueous composition is preferred, wherein:
- the at least one water-soluble or water-dispersible natural or synthetic polymer is hydroxypropylmethylcellulose, present in an amount from 2% by weight to 3% by weight, based on the total weight of the composition,
- the at least one first surfactant is polyoxyethylen(20)-sorbitan-monooleat (Tween^{®} 80), present in an amount from 0.1% by weight to 0.5% by weight, based on the total weight of the composition,
- the at least one second surfactant is sodium glycocholate (SGC), present in an amount from 1% by weight to 2% by weight, based on the total weight of the composition, and
- the at least one active pharmaceutical ingredient selected from the group of avermectins is ivermectin, present in an amount from 2% by weight to 8% by weight, based on the total weight of the composition,
- the at least one buffer is sodium citrate buffer, present in an amount from 0,14% by weight to 6% by weight, based on the total weight of the composition,
- water is present in an amount from 80% by weight to 94.7% by weight, based on the total weight of the composition, and
- the at least one active pharmaceutical ingredient has a mean particle size of 1 µm to 3 µm.

The aqueous composition mentioned above was found to be especially advantageous, concerning the adhesion properties and the viscosity properties of the inventive aqueous composition as well as the stability, solubility and uptake of an active pharmaceutical ingredient, especially with a mean particle size of 1 µm to 3 µm, from the inventive aqueous composition into the body providing a sustained release of the compound for a period of at least 3 days, preferably at least 5 days, after a single administration at organic tissue (bioavailability).

It was found that the inventive aqueous composition comprising at least one buffer is especially useful for providing a good stability of the active pharmaceutical ingredient having a mean particle size in the micron range, in particular a mean particle size of 1 µm to 3 µm.

According to the invention the rest of the aqueous composition not being a polymer, surfactant, active pharmaceutical ingredient, additives and optionally a buffer is preferably water, in particular demineralized water or purified water or even water for injection.

In a preferred embodiment of the invention water is present in the inventive aqueous composition in an amount from 60% by weight to 97.7% by weight, preferably from 80% by weight to 94% by weight, more preferably in an amount from 86% by weight to 94% by weight, based on the total weight of the composition.

The objects of this inventions are further solved by an aqueous composition comprising
- at least one water-soluble or water-dispersible natural or synthetic polymer in an amount from 0.1% by weight to 40% by weight, based on the total weight of the composition,
- at least one first surfactant in an amount from 0.1% by weight to 20% by weight, based on the total weight of the composition,
- optionally at least one second surfactant in an amount from 0.1% by weight 20% by weight, based on the total weight of the composition,
- at least one active pharmaceutical ingredient selected from the group of avermectins in an amount from 0,1% by weight to 30% by weight, based on the total weight of the composition, and
- water in an amount from 30% by weight to 99% by weight, based on the total weight of the composition.

With respect to further features and advantages of the above described aqueous composition, reference is made in its entirety to the previous description.

According to the invention the aqueous composition is preferably provided for nasal application, in particular in the form of a nasal spray.

Due to the low solubility of avermectins in aqueous media a multiparticulate inventive aqueous composition as described above was found to show an advantageous consistent application of suitable aerosolized droplets in an appropriate size range, avoiding loss of drug medication from the nasal cavity after application (to throat and/or lung) and avoiding clogging of the spray pump device by using an increased drug loading in the aqueous composition.

The invention further provides a discharging device, in particular a blow-fill-seal-ampoule, syringe or a spray device, wherein the discharging device is filled with the aqueous composition as claimed and as defined above.

In another embodiment of the invention the discharging device is preferably a CycloOlefinPolymer (COP) syringe, which shows the advantage of bearing no risk to have any residual glass particles from the manufacturing process included.

In another embodiment of the invention the discharging device is preferably a blow-fill-seal-ampoule, which is suitable for the application of low amounts for an e.g. nasal application.

In a further embodiment of the invention the discharging device, in particular the blow-fill-seal-ampoule, is preferably filled under aseptic conditions with a sterile, inventive aqueous composition.

In another embodiment of the invention the discharging device is preferably a plastic spray device, suitable for application of aqueous compositions.

Finally, the invention provides an aqueous composition as claimed and as defined above, for use in a pharmaceutical composition or a medicament, wherein preferably said use is for the prevention or treatment, in particular local treatment, of submicroscopic infectious agents that replicates only inside the living cells of an organism, so-called viral infections more preferably in the prevention or treatment of a SARS-Covid-2 infection or of a HIV infection or of a dengue virus infection.

When infected, a host cell is forced to rapidly produce thousands of identical copies of the original virus. Ivermectin has found to be active against a viral disease, the SARS-Covid 2 virus [https://www. monash.edu/discovery-institute/news-and-events/news/2020-articles/Lab-experiments-show-anti-parasitic-drug,-Ivermectin,-eliminates-SARS-CoV-2-in-cells-in-48-hours].

The inventive aqueous composition containing multiparticulates of avermectins, in particular ivermectin, has the advantage of killing virus particles locally entering the nasal cavity from the environment due to the advantageous therapeutical effect comprised by the a high concentration of the at least one active pharmaceutical ingredient selected from the group of avermectins and a large surface of the multiparticulates of the active pharmaceutical ingredient.

The inventive aqueous composition can be applied in volumes from 20-400 µl, preferably from 100-200 µl, in particular into the nasal cavity.

In a further embodiment of the invention the selected dose for a nasal treatment of a SARS-Covid-2 infection is equal or above 3 mg per nostril for ivermectin in humans.

With respect to further features and advantages of the use, in particular in terms of the aqueous composition, reference is made in its entirety to the previous description.

Further features and advantages of the invention will become clear from the following examples in conjunction with the subject-matter of the dependent claims. The individual features can be realized either singularly or in combination in one embodiment of the invention. The preferred embodiments merely serve for illustration and better understanding of the invention and are not to be understood as in any way limiting the invention.

### BRIEF FIGURE DESCRIPTION

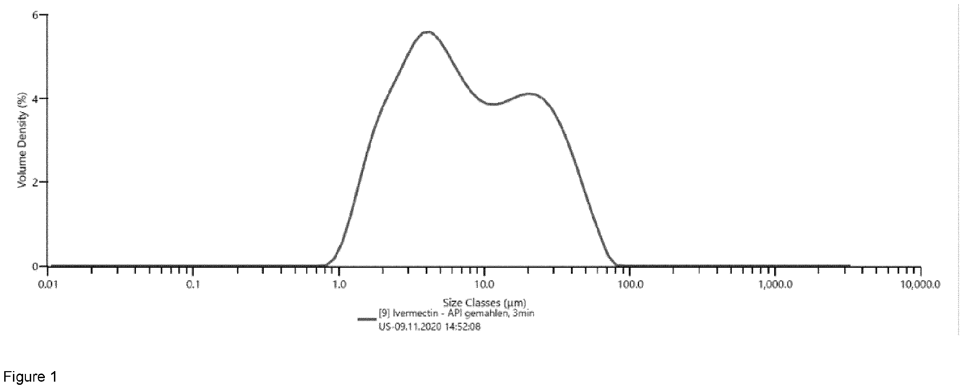

Figure 1 graphically shows PSD of grinded API (batch 05KB21.HM00155),

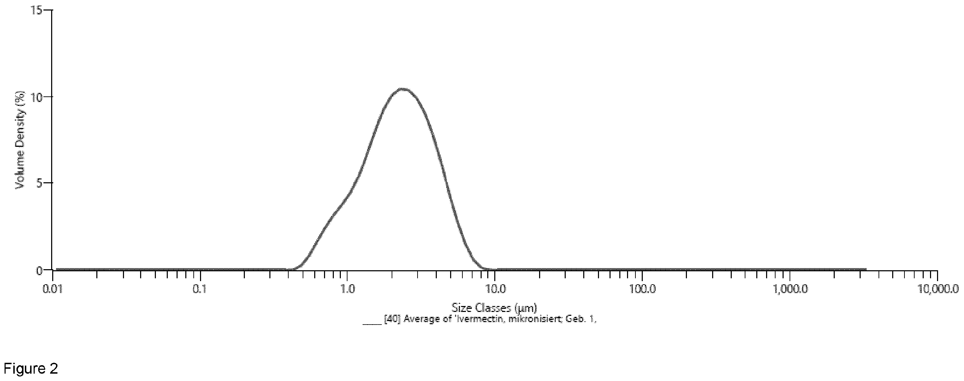

Figure 2 graphically shows PSD of micronised API (batch 05KB21.HM00155),

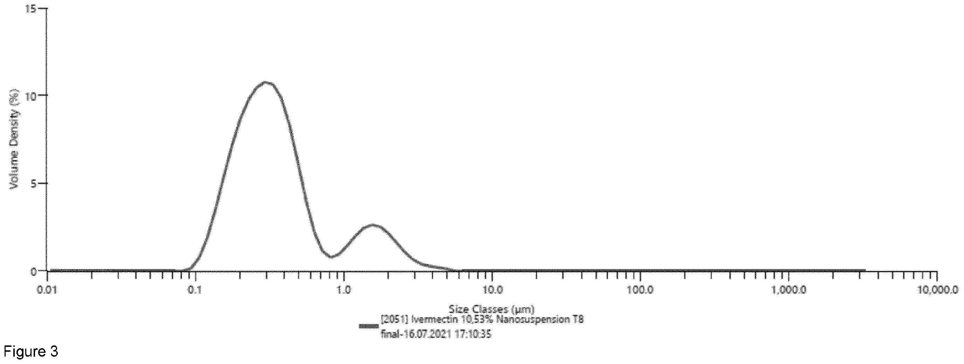

Figure 3 graphically shows PSD of wet milled Nanosuspension (batch F004-NAS-D-2107-001),

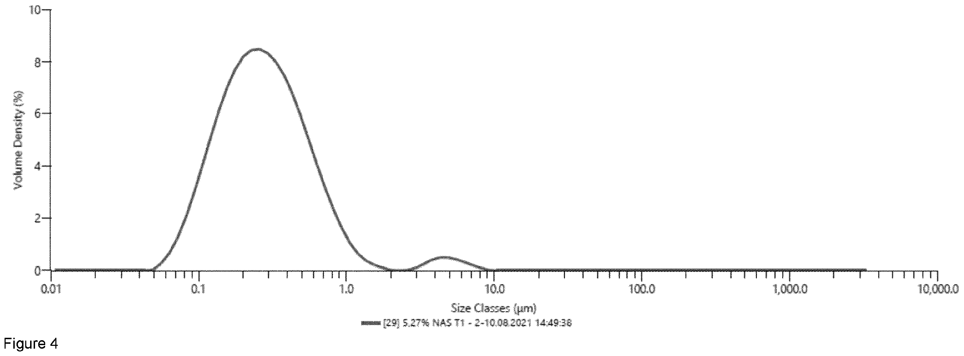

Figure 4 graphically shows PSD of wet milled Nanosuspension (batch F004-NAS-E-2108-001),

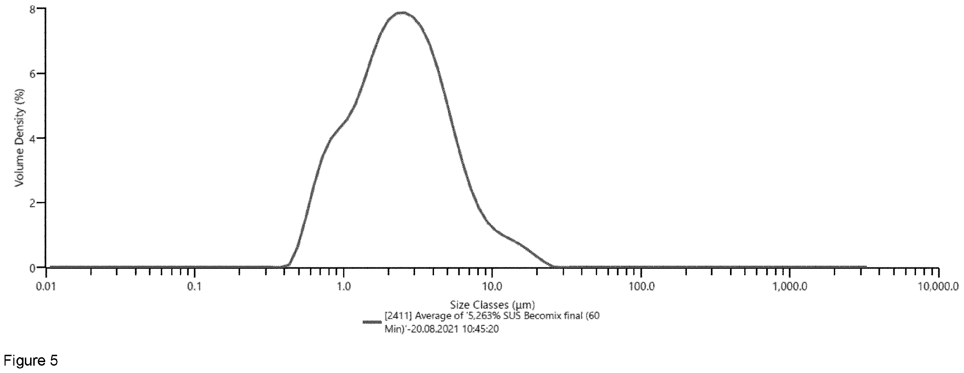

Figure 5 graphically shows PSD of bulk microsuspension (batch F004-SUS-F-2108-001),

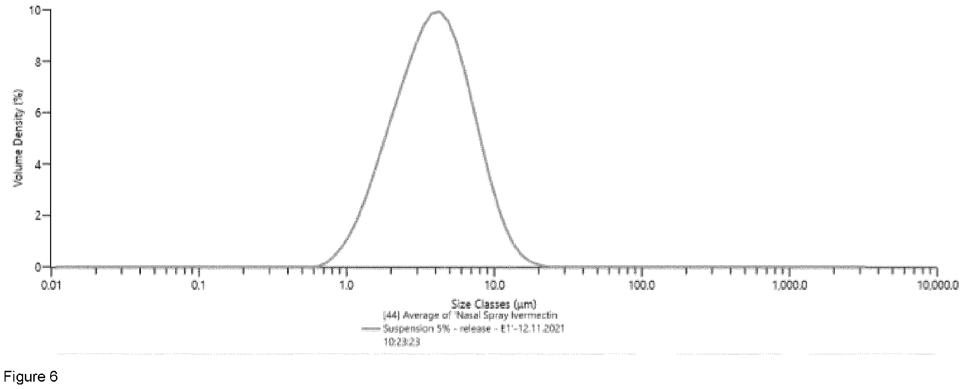

Figure 6 graphically shows PSD of final product (batch F004-SUS-G-2111-001),

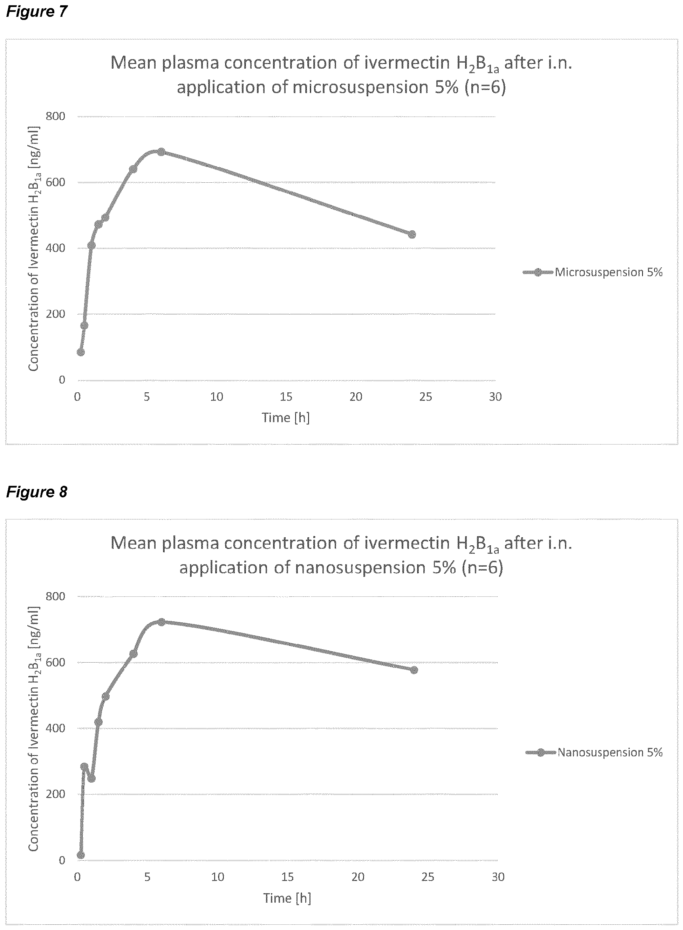

Figure 7 graphically shows pk-plasma profile of microsuspension 5% in female rats (batch F004-SUS-G-2109-001-02) and
Figure 8 graphically shows pk-plasma profile of nanosuspension 5% in female rats (batch F004-NAS-F-2109-005-03).

### EXAMPLES

Lab scale trials:
As pharmaceutically active agent ivermectin was used for the trials.

### Procedure 1: Preparation of multiparticulates of ivermectin via dry milling/grinding

The pharmaceutically active agent is grinded in a mortar/pestle system to the appropriate particles size prior to its use. Afterwards the Particles Size Distribution (PSD) is measured using a Malvern Master Sizer 3000. The samples after grinding are stored in tightly closed brown glass bottles.

### Procedure 2: Preparation of multiparticulates of ivermectin via dry milling/micronisation

The pharmaceutically active agent is micronised to the desired particles size using a jet mill (e.g. from SpiraNo 100 from MCKO, Germany) and screw dosing system (e.g. ZD 9 from Three-Tec Ltd., Seon, Switzerland). The diameter of the injection nozzle is selected at about 6 mm, the milling gas pressure about 2 bar, the milling temperature is about 18 °C. The duration of the process is about 5-10 minutes depending on the particle size of the non-micronised active agent. Afterwards the PSD is measured with a Malvern Master Sizer 3000. The samples after micronisation are stored in tightly closed brown glass bottles.

### Procedure 3: Preparation of a multiparticulate aqueous suspension of ivermectin via wet milling

The grinded pharmaceutically active agent (according to procedure 1 or 2) is weighed into a 2 ml PP (Polypropylene) tube containing about 1 g milling beads (made of yttrium stabilised zirconium oxide or comparable) of appropriate size (e.g. 0.1 mm - 0.8 mm). The aqueous solution comprising the polymer(s) and the first surfactant(s) and the second surfactant(s) in the specified amounts as stabilisers for the multiparticulate suspensions are added and the PP tube is closed. The content is mixed for some minutes and placed into a dual centrifugation system (Delta Vita 1, Netzsch, Selb, Germany or comparable). The system is started and operated under the following conditions: 1500 rpm, 1.5 hours, active cooling (≤ 25 °C). Afterwards the probes are stored overnight in order to settle eventually formed foam. The content from the PP tubes is withdrawn via a 1 ml syringe with 22-gauge needle, in order to separate the sample from the milling beads.

Afterwards the PSD is measured with a Malvern Master Sizer 3000. In addition, the Zetapotential, an index for the stability of the multiparticulate suspensions is measured using a Zeta Sizer (e.g. Nano ZS) from Malvern Instruments (Dynamic Laser diffraction).

### Procedure 4: Preparation of an aqueous composition containing ivermectin multiparticulates via wet milling

The pharmaceutically active agent is grinded/micronised (see procedures 1 and 2 above). Afterwards the grinded or micronised pharmaceutically active agent is suspended in the aqueous solution comprising polymer(s) and the first surfactant(s) and the second surfactant(s) in the specified amounts under stirring and homogenisation (e.g. by using a Becomix RW 15 CD with a stirrer speed of 10 m/s and homogenisator speed of 25 m/s or Ultraturrax, type T25 digital). The stirring is performed at 2-25 °C until a homogeneous microsuspension is obtained. Optionally, the named aqueous solution can be filtrated using a 0.22 µm pore size filter (e.g. Sartorius Sartopore 2, Capsule PES) prior to addition of the active ingredient in order to reduce the bioburden.

The suspension is then milled using a nanomill (e.g. using a VMA Getzmann, Germany (type SL 50 or comparable), in which prior to the bead milling the milling chamber (approx. 500 ml) is loaded with milling beads (made of yttrium stabilised zirconium oxide or comparable) of appropriate size (e.g. 0.1 mm - 0.8 mm). The (nano)milling process is conducted in a recirculation mode until the PSD matches the anticipated range with a mean value of about 0.1-0.5 µm. Once the anticipated PSD is reached, the process is stopped.

### Procedure 5: Preparation of an aqueous composition containing ivermectin multiparticulates - bulk microsuspension

The pharmaceutically active agent is grinded/micronised (see procedures 1 and 2 above). Afterwards the grinded or micronised pharmaceutically active agent is suspended in the aqueous solution comprising the polymer(s) and the first surfactant(s) and the second surfactant(s) in the specified amounts under stirring and homogenisation (e.g. by using a Becomix RW 15 CD with a stirrer speed of 10 m/s and homogenisator speed of 25 m/s or Ultraturrax, type T25 digital). The stirring is performed at 2-25 °C until a homogeneous microsuspension is obtained.

Optionally, the named aqueous solution can be filtrated using a 0.22 µm pore size filter (e.g. Sartorius Sartopore 2, Capsule PES) prior to addition of the active pharmaceutical ingredient in order to reduce the bioburden.

### Procedure 6: Filling of suspensions obtained from procedure 4 and 5 into appropriate devices - final product

The resulting nanosuspensions (procedure 4) or microsuspension (procedure 5) are diluted with water or the selected buffer(s) to receive the final product. Afterwards the final product is filled (e.g. with a Bausch filling system Base type 533) into plastic or glass bottles (e.g. 10 ml brown glass bottle from Nipro, hydrolytic class I) and closed with pump heads, depending on the selected device (e.g. Aptar CPS spray pump). Optionally nitrogen is used in this step as inert gas.

The following tables (see Table 1 to Table 5) give an overview of some relevant batches:

**Table 1: Lab scale suspension batches with compositions and initial PSD data**

| **Batch F004-** | **API [%]** | **Water [%]** | **Stabilizing polymers** | | | | | | **Stabilizing first/second surfactants** | | | | | | **Particle Size Distribution** | | |
|---|---|---|---|---|---|---|---|---|---|---|---|---|---|---|---|---|---|
| | | | **HPC-SL [%]** | **HPMC** 603 **[%]** | **Poloxamer** 188 **[%]** | **Poloxamer 407 [%]** | **PVP VA 64 [%]** | **PVP K30 [%]** | **DOSS [%]** | **Tween 80 [%]** | **SGC [%]** | **STC [%]** | **SDS [%]** | **TPGS [%]** | | | |
| | | | | | | | | | | | | | | | **D 10 [µm]** | **D 50 [µm]** | **D 90 [µm]** |
| NAS-A-2006-001 | 10 | 86.5 | | | | 3 | | | 0.5 | | | | | | 0.054 | 0.134 | 0.598 |
| NAS-A-2006-002 | 10 | 86.5 | | | 3 | | | | 0.5 | | | | | | 0.048 | 0.131 | 0.467 |
| NAS-A-2006-003 | 10 | 84 | | 2 | | | | | | | | | | 4 | 0.050 | 0.127 | 0.470 |
| NAS-A-2006-004 | 10 | 86 | | 2 | | | | | | | | | | 2 | 0.048 | 0.116 | 0.381 |
| NAS-A-2006-005 | 10 | 88 | | | | | | | | | | | | 2 | 0.047 | 0.113 | 0.339 |
| NAS-A-2006-007 | 10 | 86.9 | | 3 | | | | | 0.1 | | | | | | 0.057 | 0.161 | 0.642 |
| NAS-A-2006-008 | 10 | 86.9 | | | | | 3 | | 0.1 | | | | | | 0.044 | 0.107 | 0.268 |
| NAS-A-2006-009 | 10 | 86.9 | | | | | 3 | | | | | | 0.1 | | 0.041 | 0.105 | 0.263 |
| NAS-A-2006-010 | 10 | 85.5 | | | | | 3 | | | 0.5 | | | | 1 | 0.049 | 0.117 | 0.408 |

Synonyms: HPC-SL (=Hydroxypropylcellulose SL); HPMC 603 (=Hydroxypropylmethylcellulose 603); PVP VA 64 (=Polyvinylpyrrolidon Vinaylacetate copolymer 64); PVP K30 (=Polyvinylpyrrolidon K30); DOSS (=Dioctylsulfosuccinat ester); Tween 80 (= Polysorbate 80); SGC =( Sodiumglycocholate) ; STC (=Sodium taurocholate); SDS =( Sodium dodecyl sulfate) ; TPGS ( = D-alpha tcocopherol polyethylene glycol 1000 succinat)

**Table 2: Lab scale suspension batches with compositions and initial PSD data**

| **Batch F004-** | **API [%]** | **Water [%]** | **Stabilizing polymers** | | | | | | **Stabilizing first/second surfactants** | | | | | | **Particle Size Distribution** | | |
|---|---|---|---|---|---|---|---|---|---|---|---|---|---|---|---|---|---|
| | | | **HPC-SL [%]** | **HPMC 603 [%]** | **Poloxamer 188 [%]** | **Poloxamer 407 [%]** | **PVP VA 64 [%]** | **PVP K30 [%]** | **DOSS [%]** | **Tween 80 [%]** | **SGC [%]** | **STC [%]** | **SDS [%]** | **TPGS [%]** | | | |
| | | | | | | | | | | | | | | | **D 10 [µm]** | **D 50 [µm]** | **D 90 [µm]** |
| NAS-A-2006-011 | 10 | 86.9 | | 3 | | | | | | | | | 0.1 | | 0.044 | 0.110 | 0.280 |
| NAS-A-2006-012 | 10 | 86.5 | | 3 | | | | | | | | | 0.5 | | 0.039 | 0.099 | 0.235 |
| NAS-A-2006-013 | 10 | 86.5 | 3 | | | | | | 0.5 | | | | | | 0.044 | 0.110 | 0.271 |
| NAS-A-2006-014 | 10 | 86.9 | 3 | | | | | | 0.1 | | | | | | 0.043 | 0.105 | 0.261 |
| NAS-A-2006-015 | 10 | 83.9 | 6 | | | | | | 0.1 | | | | | | 0.033 | 0.136 | 0.666 |
| NAS-A-2006-016 | 10 | 86.5 | | | | | 3 | | | | | | 0.5 | | 0.040 | 0.102 | 0.247 |
| NAS-A-2006-018 | 10 | 86.9 | | | 3 | | | | | | | | 0.1 | | 0.044 | 0.108 | 0.274 |
| NAS-A-2006-01 | 10 | 86.5 | | | 3 | | | | | | | | 0.5 | | 0.048 | 0.116 | 0.359 |
| NAS-A-2006-020 | 10 | 85 | | | 3 | | | | | | | | | 2 | 0.051 | 0.122 | 0.532 |
| NAS-A-2006-021 | 10 | 83.5 | | | | | 6 | | 0.5 | | | | | | 0.039 | 0.098 | 0.232 |
| NAS-A-2006-022 | 10 | 86.5 | | 3 | | | | | | | 0.5 | | | | 0.051 | 0.127 | 0.332 |
| NAS-A-2006-023 | 10 | 83.5 | | 6 | | | | | | | 0.5 | | | | 0.110 | 0.412 | 11.30 |
| 125 | 20 | 76.0 | | | 3 | | | | | | 1 | | | | 0.047 | 0.111 | 0.315 |
| 126 | 20 | 73.5 | | | 4.5 | | | | | | | | | 2 | 0.079 | 0.211 | 0.816 |
| NAS-C-2102-006 | 15 | 81.0 | | 2 | | | | | | | 2 | | | | 0.070 | 0.150 | 0.340 |

Synonyms: HPC-SL (=Hydroxypropylcellulose SL); HPMC 603 (=Hydroxypropylmethylcellulose 603); PVP VA 64 (=Polyvinylpyrrolidon Vinaylacetate copolymer 64); PVP K30 (=Polyvinylpyrrolidon K30); DOSS (=Dioctylsulfosuccinat ester); Tween 80 (= Polysorbate 80); SGC =( Sodiumglycocholate) ; STC (=Sodium taurocholate); SDS =( Sodium dodecyl sulfate) ; TPGS ( = D-alpha tcocopherol polyethylene glycol 1000 succinat)

**Table 3: Lab scale suspension batches with compositions and initial PSD data**

| **Batch F004-** | **API concentration [%]** | **Stabilizing Polymer** | **Stabilizing first surfactant** | **Stabilizing second surfactant** | **Particle Size Distribution** | | |
|---|---|---|---|---|---|---|---|
| | | | | | **D 10 [µm]** | **D 50 [µm]** | **D 90 [µm]** |
| NAS-C-2101-003 | 15 | 2 % HPMC 603 | - | 1 % SGC | 0.215 | 0.353 | 0.563 |
| NAS-C-2101-004 | 15 | 1 % HPMC 603 | - | 1 % SGC | 0.240 | 0.383 | 0.624 |
| NAS-C-2101-005 | 15 | 2 % HPMC 603 | - | 0.5 % SGC | 0.277 | 0.477 | 2.14 |
| NAS-C-2101-006 | 15 | 2 % HPMC 603 | - | 2 % SGC | 0.191 | 0.325 | 0.535 |
| NAS-C-2101-003 | 15 | 2 % HPMC 603 | - | 1 % SGC | 0.215 | 0.353 | 0.563 |
| NAS-C-2102-009 | 15 | 3 % HPC SL | - | 2 % SGC | 0.199 | 0.337 | 0.546 |
| NAS-E-2108-003-01 | 5.27 | 3.16 % PVP PF 17 | 0.32 % Tween 80 | 1.05 % SGC | 0.156 | 0.267 | 0.444 |
| NAS-G-2108-001-01 | 5.32 | 3.19 % Lipoid E 80 | 0.32 % Tween 80 | 1.06 % SGC | 0.161 | 0.281 | 0.484 |
| NAS-G-2108-002-01 | 5.32 | 3.19 % Lipoid E 80 | - | 1.06 % SGC | 0.144 | 0.243 | 0.409 |
| NAS-F-2108-005-02 | 5 | 3 % PVP PF 17 | 0.3 % Tween 80 | 1 % SGC | 0.264 | 0.415 | 0.672 |
| NAS-F-2108-007-02 | 5 | 2 % HPMC 603 | 0.3 % Tween 80 | 1 % SGC | 0.296 | 0.481 | 0.911 |
| | | 3 % PVP PF 17 | | | | | |

**Table 4: Zeta potential (measured with Malvern Zeta Sizer Nano ZS) of selected lab scale batches**

| **Batch F004** | **API concentration [%]** | **Stabilizing Polymer(s)** | **Stabilizing first/second surfactants** | **Zetapotential [mV]** |
|---|---|---|---|---|
| \|25 | 20 | 3 % HPMC 603 | 1 % SGC | -31.2 |
| \|26 | 20 | 4.5 % Poloxamer 188 | 2 % TPGS | -31.8 |
| NAS-C-2102-006 | 15 | 2 % HPMC 603 | 2 % SGC | -31.0 |
| NAS-F-2108-007-02 | 5 | 2 % HPMC 603 | 1 % SGC | n.a. |
| | | 3 % PVP PF 17 | 0.3 % Tween 80 | |

**Table 5: Lab scale suspension batches with PSD (initial and after a storage period of 2 weeks at 20-25 °C)**

| **Batch F004-** | **API concentration [%]** | **PSD (initial) [µm]** | | | **PSD (after 2 weeks at 20-25 °C) [nm]** | | |
|---|---|---|---|---|---|---|---|
| | | **D 10 [µm]** | **D 50 [µm]** | **D 90 [µm]** | **D 10 [µm]** | **D 50 [µm]** | **D 90 [µm]** |
| NAS-A-2006-001 | 10 | 0.054 | 0.134 | 0.598 | 0.043 | 0.112 | 0.306 |
| NAS-A-2006-002 | 10 | 0.048 | 0.131 | 0.467 | 0.051 | 0.127 | 0.465 |
| NAS-A-2006-003 | 10 | 0.050 | 0.127 | 0.470 | 0.048 | 0.116 | 0.367 |
| NAS-A-2006-005 | 10 | 0.047 | 0.113 | 0.339 | 0.048 | 0.115 | 0.354 |
| NAS-A-2006-007 | 10 | 0.057 | 0.161 | 0.642 | 0.044 | 0.108 | 0.282 |
| NAS-A-2006-00 | 10 | 0.044 | 0.107 | 0.268 | 0.043 | 0.106 | 0.263 |
| NAS-A-2006-009 | 10 | 0.041 | 0.105 | 0.263 | 0.041 | 0.103 | 0.247 |
| NAS-A-2006-010 | 10 | 0.049 | 0.117 | 0.408 | 0.044 | 0.107 | 0.277 |
| NAS-A-2006-011 | 10 | 0.044 | 0.110 | 0.28 | 0.043 | 0.107 | 0.27 |
| NAS-A-2006-014 | 10 | 0.043 | 0.105 | 0.261 | 0.042 | 0.104 | 0.258 |
| NAS-A-2006-016 | 10 | 0.040 | 0.102 | 0.247 | 0.040 | 0.102 | 0.247 |
| NAS-A-2006-01 | 10 | 0.044 | 0.108 | 0.274 | 0.045 | 0.110 | 0.273 |
| NAS-A-2006-020 | 10 | 0.051 | 0.122 | 0.532 | 0.043 | 0.105 | 0.266 |
| NAS-A-2006-021 | 10 | 0.039 | 0.098 | 0.232 | 0.040 | 0.096 | 0.224 |
| NAS-A-2006-022 | 10 | 0.051 | 0.127 | 0.332 | 0.050 | 0.125 | 0.322 |
| NAS-C-2102-006 | 15 | 0.199 | 0.337 | 0.546 | 0.175 | 0.302 | 0.493 |
| NAS-F-2108-007-02 | 5 | 0.296 | 0.481 | 0.911 | 0.286^{∗} | 0.466^{∗} | 0.896^{∗} |

| | | | | | | | |
|---|---|---|---|---|---|---|---|
| ^{∗} *after 6 days* | | | | | | | |

Examples of representative batch compositions for procedure 4 are described in Table 6 and Table 7.

**Table 6: Batch composition in accordance with procedure 4, batch F004-NAS-D-2107-001**

| **Pos. No.** | **Material** | **Function** | **Concentration [%]** | **Quantitative Composition [g]** |
|---|---|---|---|---|
| 01 | Ivermectin | API | 10.53 | 105.30 |
| 02 | Tween 80 | First surfactant | 0.32 | 3.20 |
| 02 | HPMC 603 | Polymer | 2.11 | 21.10 |
| 04 | SGC | Second surfactant | 1.05 | 10.50 |
| 05 | Water | Medium | 85.99 | 859.90 |
| **Sum** | | | **100.00** | **1000.00** |

**Table 7: Batch composition in accordance with procedure 4, batch F004-NAS-E-2108-001**

| **Pos. No.** | **Material** | **Function** | **Concentration [%]** | **Quantitative Composition [g]** |
|---|---|---|---|---|
| 01 | Ivermectin | API | 5.27 | 52.70 |
| 02 | Tween 80 | First surfactant | 0.32 | 3.20 |
| 03 | HPMC 603 | Polymer | 2.11 | 21.10 |
| 04 | SGC | Second surfactant | 1.05 | 10.50 |
| 05 | Water | Medium | 91.25 | 912.50 |
| **Sum** | | | **100.00** | **1000.00** |

Examples of representative batch compositions for procedure 5 are described in Table 8 and Table 9.

**Table 8: Batch composition in accordance with procedure 5, batch F004-SUS-E-2107-001**

| **Pos. No.** | **Material** | **Function** | **Concentration [%]** | **Quantitative Composition [g]** |
|---|---|---|---|---|
| 01 | Ivermectin | API | 10.52 | 210.40 |
| 02 | Tween 80 | First surfactant | 0.32 | 6.40 |
| 03 | HPMC 603 | Polymer | 2.11 | 42.20 |
| 04 | SGC | Second surfactant | 1.05 | 21.00 |
| 05 | Water | Medium | 86.00 | 1720.00 |
| **Sum** | | | **100.00** | **2000.00** |

**Table 9: Batch composition in accordance with procedure 5, batch F004-SUS-F-2108-001**

| **Pos. No.** | **Material** | **Function** | **Concentration [%]** | **Quantitative Composition [g]** |
|---|---|---|---|---|
| 01 | Ivermectin | API | 5.26 | 150.00 |
| 02 | Tween 80 | First surfactant | 0.32 | 9.00 |
| 03 | HPMC 603 | Polymer | 2.11 | 60.00 |
| 04 | SGC | Second surfactant | 1.05 | 30.00 |
| 05 | Water | Medium | 91.26 | 2601.00 |
| **Sum** | | | **100.00** | **2850.00** |

Examples of representative batch compositions for procedure 6 are shown in Table 10 and Table 11.

**Table 10: Batch composition of exemplary batch F004-NAS-F-2110-009-02 (nanosuspension according to procedure 6)**

| **Pos. No.** | **Material** | **Function** | **Concentration [%]** | **Quantitative Composition [g]** |
|---|---|---|---|---|
| 01 | Ivermectin | API | 5.00 | 250.00 |
| 02 | Tween 80 | First surfactant | 0.30 | 15.00 |
| 03 | Hydroxypropylmethylcellulose | Polymer | 2.00 | 100.00 |
| 04 | Sodium glycocholate | Second surfactant | 1.00 | 50.00 |
| 05 | Water | Medium | 91.70 | 4585.00 |
| **Sum** | | | **100.00** | **5000.00** |

**Table 11: Batch composition of exemplary batch F004-SUS-G-2111-001 (microsuspension according to procedure 6)**

| **Pos. No.** | **Material** | **Function** | **Concentration [%]** | **Quantitative Composition [g]** |
|---|---|---|---|---|
| 01 | Ivermectin | API | 5.00 | 250.00 |
| 02 | Tween 80 | First surfactant | 0.30 | 15.00 |
| 03 | Hydroxypropylmethylcellulose | Polymer | 2.00 | 100.00 |
| 04 | Sodium glycocholate | Second surfactant | 1.00 | 50.00 |
| 05 | Citrate buffer pH 6.2 | Buffer | 5.00 | 250.00 |
| 06 | Water | Medium | 86.70 | 4335.00 |
| **Sum** | | | **100.00** | **5000.00** |

### Rheological measurements

Viscosities are measured using an Anton Paar Rheometer. A shear speed of 50 1/s with constant rotation is used. A suitable measurement time is e.g. up to 10 min. As measuring temperatures about 20 °C and about 35 °C are used.

For preparing the sample and the measurement, the rheometer is tempered to the desired measurement temperature via a thermostat. Then, the sample (1 ml) is added dropwise on the sample plate using a scaled pipette, Eppendorf Pipette or a suitable syringe to achieve a complete filling of the measuring gap of the rheometer. After removing excess sample material, the sample is also tempered to the desired temperature and the measurement is conducted.

In some embodiments the viscosity is measured using the above described procedure. The following Table *12* shows viscosity measurements of one exemplary batch F004-SUS-G-2018-001 at 20 and 35 °C.

**Table 12: Viscosity measurements of exemplary batch and F004-SUS-G-2018-001**

| **Batch F004- SUS-G-2018-001** | **Composition [%]** |
|---|---|
| Ivermectin | 5.0 |
| Tween 80 | 0.3 |
| HPMC 603 | 2.0 |
| SGC | 1.0 |
| Citrate buffer (pH 6.2) | 5.0 |
| **Viscosity [mPas] at 20 °C** | **5.414** |
| **Viscosity [mPas] at 35 °C** | **4.664** |

### Particle size distribution (PSD)

In some embodiments, the PSD of the pharmaceutically active agent, provided for the preparation of its multiparticulate suspension according to procedure 1 and procedure 2 (grinded/micronised), was measured using a Malvern Mastersizer 2000 or 3000 (in water as dispersion medium, stirrer speed 1500 rpm, Mie presentation) to give the following results:

**Table 13: PSD of API batch after grinding**

| **API Batch** | **D10 [µm]** | **D50 [µm]** | **D90 [µm]** |
|---|---|---|---|
| 05KB21.HM00155 | 2.02 | 6.89 | 32.0 |

**Table 14: PSD of API batch after micronisation**

| **API Batch** | **D10 [µm]** | **D50 [µm]** | **D90 [µm]** |
|---|---|---|---|
| 05KB21.HM00170-mic | 0.97 | 2.22 | 4.35 |

In some embodiments, the PSD of the pharmaceutically active agent in multiparticulate suspension prepared according to procedure 3 (wet ball milling with Delta Vita 1), procedure 4 (wet ball milling Getzmann/Frewitt), procedure 5 (bulk microsuspension) and procedure 6 (final product) was measured using a Malvern Mastersizer 2000 or 3000 (in water as dispersion medium, stirrer speed 1500 rpm, Mie presentation).

In the following Table *15*, results for exemplary batches are shown.

**Table 15: PSD data of batches in accordance to the described procedures**

| **Batch F004-** | **Remark** | **D10 [µm]** | **D50 [µm]** | **D90 [µm]** |
|---|---|---|---|---|
| NAS-D-2107-001 | 10% Nanosuspension bulk | 0.17 | 0.32 | 1.42 |
| NAS-E-2108-001 | 5% Nanosuspension bulk | 0.115 | 0.27 | 0.68 |
| SUS-E-2017-001 | 10% Microsuspension bulk | 1.10 | 2.84 | 6.93 |
| SUS-F-2018-001 | 5% Microsuspension bulk | 0.87 | 2.39 | 6.29 |
| SUS- E-2107-001 | 10% final product | 1.12 | 2.84 | 6.93 |
| SUS-G-2111-001 | 5% final product | 1.70 | 3.84 | 8.02 |

### Stability

### In another embodiment, the stability of the inventive aqueous compositions was investigated. The results are reported exemplarily in the following

Table *17* and Table *18* for batches manufactured in accordance with procedure 6, containing ivermectin as the pharmaceutically active agent. The compositions are stated in Table *16*.

**Table 16: Compositions of exemplary batches used for PSD, osmolarity, pH and purity via HPLC investigation**

| **Batch F004-** | **Ivermectin [%]** | **Tween 80 [%]** | **HPMC 603 [%]** | **SGC [%]** | **Citrate buffer pH 6.2 [%]** | **BHT [%]** | **Water [%]** |
|---|---|---|---|---|---|---|---|
| SUS-B-2107-040 | 10 | 0.3 | 2.0 | 1.0 | - | - | 86.70 |
| SUS-B-2107-038 | 10 | 0.3 | 2.0 | 1.0 | - | 0.01 | 86.69 |
| SUS-B-2107-037 | 10 | 0.3 | 2.0 | 1.0 | 5.0 | - | 81.7 |
| SUS-B-2107-039 | 10 | 0.3 | 2.0 | 1.0 | 4.99 | 0.01 | 81.7 |

**Table 17: Analytical results of exemplary microsuspensions (according to procedure 6, non-buffered) tested for PSD, osmolarity, pH and purity via HPLC after being stored for 2 and 4 weeks at 40 °C**

| **Batch F004-** | SUS-B-2107-040 | SUS-B-2107-040 | SUS-B-2107-038* | SUS-B-2107-040 | SUS-B-2107-038 |
|---|---|---|---|---|---|
| **Storage period** | Initial | 2 weeks at 40 °C | 2 weeks at 40 °C | 4 weeks at 40 °C | 4 weeks at 40 °C |
| **PSD [µm] d10/d50/d90** | 0.89/2.50/7.49 | 0.85/2.40/6.54 | n.a. | 0.84/2.41/6.71 | 0.83/2.39/6.60 |
| **Osmolarity [mOsmol/kg]** | 117 | 114 | n.a. | 114 | n.a. |
| **pH** | 6.4 | 5.8 | n.a. | 5.5 | n.a. |
| **Purity** Σ **[%]** | 4.72 | 4.89 | 5.14 | 5.20 | 5.48 |

| | | | | | |
|---|---|---|---|---|---|
| **has not been tested initial* | | | | | |

**Table 18: Analytical results of exemplary microsuspensions (according to procedure 6, buffered) tested for PSD, osmolarity, pH and purity via HPLC after being stored for 2 and 4 weeks at 40 °C**

| **Batch F004-** | SUS-B-2107-037 | SUS-B-2107-037 | SUS-B-2107-039* | SUS-B-2107-037 | SUS-B-2107-040 |
|---|---|---|---|---|---|
| **Storage period** | Initial | 2 weeks at 40 °C | 2 weeks at 40 °C | 4 weeks at 40 °C | 4 weeks at 40 °C |
| **PSD [µm] d10/d50/d90** | 0.89/2.47/6.70 | 1.44/3.15/6.79 | n.a. | 1.38/2.89/5.92 | 1.25/2.62/5.68 |
| **Osmolarity [mOsmol/kg]** | 250 | 246 | n.a. | 233 | n.a. |
| **pH** | 6.7 | 6.7 | n.a. | 6.6 | n.a. |
| **Purity** Σ **[%]** | 4.48 | 4.55 | 4.62 | 4.63 | 4.55 |

| | | | | | |
|---|---|---|---|---|---|
| **has not been tested initial* | | | | | |

### Osmolarity

In some embodiments, the Osmolarity was measured for the aqueous compositions by using an Osmometer (Knauer), type K 7400 with the following conditions:
Freeze: -5.5 °C, Cooling limit: -14.5 °C

### pH

In some embodiments, the pH value was measured for the aqueous compositions by using a pH meter (Mettler Toledo), type SevenExcellence.

### Shot weight

For the determination of the shot weight the following procedure was used:
Before starting the investigation of shot weight, the nasal spray is gently shaken, and three actuations are performed in order to prime the unit. The device is weighed before and after each actuation to be tested, and the emitted mass per actuation is determined.

### Droplet Size Distribution (DSD)

In another embodiment, the droplet size distribution of the inventive aqueous compositions was investigated. The determination of the droplet size was determined in accordance with the EMA GUIDELINE ON THE PHARMACEUTICAL QUALITY OF INHALATION AND NASAL PRODUCTS from 21 June 2006. A Sympathec Helos BR/Multirange system was used. The measurement parameters are stated below. Investigation is conducted over the container life (beginning, middle, end).

Results are reported exemplarily in the following
Table 20 and Table 21 for batches manufactured in accordance with procedure 6, containing ivermectin as the pharmaceutically active agent. The compositions are stated in Table *19*.

**Table 19: Compositions of exemplary batches used for DSD and shot weight investigation**

| **Batch F004-** | **Ivermectin [%]** | **Tween 80 [%]** | **HPMC 603 [%]** | **SGC [%]** | **Citrate buffer pH 6.2 [%]** | **Water [%]** |
|---|---|---|---|---|---|---|
| SUS-B-2107-037* | 10 | 0.3 | 2.0 | 1.0 | 5.0 | 81.7 |
| NAS-A-2107-039** | 10 | 0.3 | 2.0 | 1.0 | 5.0 | 81.7 |
| NAS-E-2108-001** | 5.27 | 0.32 | 2.11 | 1.05 | - | 91.3 |
| SUS-G-2111-001* | 5 | 0.3 | 2.0 | 1.0 | 5.0 | 86.7 |

| | | | | | | |
|---|---|---|---|---|---|---|
| **microsuspensions,* ***nanosuspensions* | | | | | | |

Device: 5 ml amber glass vial, hydrolytic class I, with CPS spray pump Aptar (140 µl per actuation) including air inlet filter with 0.2 µm pore diameter

**Helos system:**

| | |
|---|---|
| Distance nozzle - laser beam: | 5 cm |
| Data collection: | 200 ms |
| Spraying angle: | 45° |
| Actuation force: | 60 N |

**Table 20: Droplet Size Distribution and shot weight for compositions (in accordance with procedure 6) containing ivermectin**

| **Batch F004-** | SUS-B-2107-037 | NAS-A-2107-039 |
|---|---|---|
| **DSD [µm]** | *Initial* | *Initial* |
| **d10/d50/d90** | d10: 19.2 - 23.6 (n=3) | d10: 26.7 - 28.2 (n=3) |
| | d50: 72.4 - 148 (n=3) | d50: 94.1 - 98.9 (n=3) |
| | d90: 323 - 356 (n=3) | d90: 319 - 337 (n=3) |
| | *Middle* | *Middle* |
| | d10: 20.7 - 29.6 (n=3) | d10: 24.9 - 26.9 (n=3) |
| | d50: 77.5 - 142.8 (n=3) | d50: 95.4 - 108 (n=3) |
| | d90: 310 - 360 (n=3) | d90: 313 - 338 (n=3) |
| | *End* | *End* |
| | d10: 28.6 - 32.8 (n=3) | d10: 23.8 - 26.5 (n=3) |
| | d50: 152 - 161 (n=3) | d50: 91.1 - 111 (n=3) |
| | d90: 348 - 361 (n=3) | d90: 294 - 336 (n=3) |
| **Shot weight [mg]** | *Initial* | *Initial* |
| | 124.7 - 146.5 (n=5) | 141.9 - 144.7 (n=5) |
| | *Middle* | *Middle* |
| | 98.8 - 146.3 (n=7) | 137.4 - 148.1 (n=7) |
| | *End* | *End* |
| | 133.5 - 143.7 (n=2) | 138.7 - 142.5 (n=2) |

Device: 5 ml amber glass vial, hydrolytic class I, with CPS spray pump Aptar (140 µl per actuation) including air inlet filter with 0.2 µm pore diameter

**Helos system:**

| | |
|---|---|
| Distance nozzle - laser beam: | 3 cm |
| Data collection: | 200 ms |
| Spraying angle: | 45° |
| Actuation force: | 60 N |

**Table 21: Droplet Size Distribution and shot weight for compositions (in accordance to procedure 6) containing ivermectin**

| **Batch F004-** | NAS-E-2018-001 | SUS-G-2111-001 |
|---|---|---|
| **DSD [µm]** | *Initial* | *Initial* |
| **d10/d50/d90** | d10: 21.8 - 22.6 (n=3) | d10: 22.0 - 22.3 (n=2) |
| | d50: 92.8 - 97.4 (n=3) | d50: 108.4 - 124.5 (n=2) |
| | d90: 320 - 332 (n=3) | d90: 324 - 258 (n=2) |
| | *Middle* | *Middle* |
| | d10: 23.0 - 23.3 (n=3) | d10: 25.6 - 26.2 (n=2) |
| | d50: 88.8 - 98.3 (n=3) | d50: 117.1 - 135.5 (n=2) |
| | d90: 306 - 314 (n=3) | d90: 343 - 349 (n=2) |
| | *End* | *End* |
| | d10: 22.2 - 23.7 (n=3) | d10: 20.9 - 22.5 (n=2) |
| | d50: 87.8 - 108 (n=3) | d50: 111.4 - 114.9 (n=2) |
| | d90: 291 - 347 (n=3) | d90: 346 - 354 (n=2) |
| **Shot weight [mg]** | *Initial* | *Initial* |
| | 144.7 - 146.4 (n=5) | 141.2 - 145.1 (n=3) |
| | *Middle* | *Middle* |
| | 131.0 - 145.3 (n=7) | 143.4 - 145.4 (n=4) |
| | *End* | *End* |
| | 141.3 - 142.7 *(n=2)* | 143.3 - 144.1 (n=3) |

### pk-study in rats

In another embodiment, the aqueous compositions were tested in a pk-study in rats and the AUC (Area Under the Curve), the tₘₐₓ and Cₘₐₓ have been determined in the blood plasma for ivermectin.

**Table 22: Compositions batches used for the pk-study in rats**

| **Batch F004-** | **Ivermectin [%]** | **Tween 80 [%]** | **HPMC 603 [%]** | **PVP 17 PF [%]** | **SGC [%]** | **Citrate buffer pH 6.2 [%]** | **Water [%]** |
|---|---|---|---|---|---|---|---|
| SUS-G-2109-001-02* | 5 | 0.3 | 2.0 | | 1.0 | 5.0 | 86.7 |
| NAS-F-2109-005-03** | 5 | 0.3 | - | 3.0 | 1.0 | - | 90.7 |
| SOL-P-B-2109-001-01*** | - | 0.3 | 2.0 | - | 1.0 | 5.0 | 91.7 |

| | | | | | | | |
|---|---|---|---|---|---|---|---|
| **microsuspensions,* ***nanosuspensions, ***vehicle (Microsuspension without active pharmaceutical ingredient)* | | | | | | | |

**Table 23: pk data for the microsuspension containing 5% Ivermectin**

| **Parameter** | **Tₘₐₓ** | **Cₘₐₓ** | **AUCₗₐₛₜ** | **MRTₗₐₛₜ** |
|---|---|---|---|---|
| **N** | 6 | 6 | 6 | 6 |
| **Mean** | 8.667 | 733.380 | 13011.911 | 11.905 |
| **SD** | 7.554 | 224.583 | 3062.972 | 1.234 |
| **CV%** | 87.2 | 30.6 | 23.5 | 10.4 |
| **Min** | 4.00 | 471.95 | 9340.50 | 10.28 |
| **Median** | 6.00 | 691.36 | 13373.35 | 11.91 |
| **Max** | 24.00 | 1088.49 | 17489.98 | 13.56 |
| **Geometric Mean** | 7.066 | 705.788 | 12707.598 | 11.852 |
| **Geometric CV%** | 68.55 | 30.97 | 24.35 | 10.45 |

**Table 24: pk data for the nanosuspension containing 5% Ivermectin**

| **Parameter** | **Tmax** | **Cmax** | **AUCₗₐₛₜ** | **MRTₗₐₛₜ** |
|---|---|---|---|---|
| **N** | 6 | 6 | 6 | 6 |
| **Mean** | 10.750 | 869.712 | 14620.814 | 12.612 |
| **SD** | 10.458 | 303.751 | 3767.716 | 1.271 |
| **CV%** | 97.3 | 34.9 | 25.8 | 10.1 |
| **Min** | 0.50 | 567.25 | 10054.00 | 11.12 |
| **Median** | 6.00 | 764.08 | 14205.90 | 12.26 |
| **Max** | 24.00 | 1317.35 | 20897.70 | 14.74 |
| **Geometric Mean** | 5.883 | 828.700 | 14233.813 | 12.560 |
| **Geometric CV%** | 258.08 | 34.57 | 25.65 | 9.86 |

### pk-Study outline

Animals: female wistar rats
Number of groups: 2 verum and 1 placebo
Batches:
   F004-SUS-G-2109-001-02 and F004-NAS-F-2109-005-03 (microsuspension and nanosuspension containing 5% ivermectin prepared in accordance with procedure 6); F004-SOL-P-B-2109-001-01 (microsuspension vehicle)
Application: intranasal with sterile pipette into anesthetized rats
Application volume: 2x 25 µl per nostril

Blood sampling: sampling from catheter in the vena jugularis; approx. 0.25 mL blood are collected into plastic vials containing K3-EDTA as anticoagulant at the following time points after administration from the catheter: 15 min, 0.5h, 1h, 1.5h, 2h, 4h, 6h and 24h; altogether 8 samples/animal.

Plasma preparation: cooling of blood samples in ice-water bath and centrifugation within 60 minutes after collection (2000 g at +4 °C for 10 min), then plasma separation Storage of plasma: the supernatant approx. 100 µL transfer pre-labelled plastic reaction tubes (e.g. Eppendorf), immediately frozen in dry-ice, storage in an ultra-freezer (below -70 °C) until transport.

Plasma analysis: The determination of ivermectin H₂B₁ₐ in rat plasma was done via LC-MS/MS after liquid-liquid extraction with Novum SLE plates. Doramectin was used as internal standard. The analytical testing method was validated under experimental conditions with a lower limit of quantification (LLOQ) of 5 ng/ml and a linear calibration range of 5-500 ng/ml for ivermectin H₂B₁ₐ. Plasma samples for which a high ivermectin H₂B₁ₐ concentration was expected, were diluted using blank plasma. The following equipment has been used:
▪ Mass spectrometer Triple Quad 6500+, Sciex equipped with a TurbolonSpray Ion source (A843-MS).
▪ LC system composed by binary pump Agilent 1290 Infinity, column oven Agilent 1290TCC and PAL HTC xt autosampler (A844-LC, A845-AS).
▪ Software Analyst 1.7.0, ABSciex
▪ Software: ANALYST (Sciex) LC-MS/MS software (version 1.6.3)

**Chromatographic conditions**

| HPLC-column: | XBridge C18, 3.5 µm, | | 100x2.1 mm, Waters | |
|---|---|---|---|---|
| | Flow rate: | | 500 µL/min | |
| HPLC-conditions: | Run time: 5 min | | | |
| | Autosampler temp.: | | 15 °C | |
| | Column heater: | | 35 °C | |
| | Injection volume: | | 5 µL | |
| Mobile phase: | A: H₂O (0.1% NH₄OH) | | | |
| | B: MeOH | | | |
| Autosampler rinsing solvents: | Wash 1: ACN/IPA/H₂O 40:40:20 v/v/v (0.2% Acetic Acid) | | | |
| | Wash 2: H₂O:MeOH 1:1 v/v | | | |
| Gradient: | **Time [min]** | **Eluent A [%]** | | **Eluent B [%]** |
| 0.0 | | 20 | | 80 |
| 2.0 | | 2 | | 98 |
| 3.5 | | 2 | | 98 |
| 3.6 | | 20 | | 80 |
| 5.0 | | 20 | | 80 |

**Detection: MRM (multiple reaction monitoring)**

| **MS Parameters:** | |
|---|---|
| lonisation Mode: | Turbo ion spray (TIS) |
| Scan type: | MRM, negative |
| Curtain Gas Flow (CUR): | 40.0 |
| Nebulizer Gas Flow (GS1): | 45 psi |
| Turbo Ion Spray Gas (GS2): | 60 psi |
| Heated Nebulizer Temp (TEM): | 500 °C |
| Collision Gas Flow (CAD): | 7.0 |
| Ion Spray Voltage (IS): | -4500 V |

Subsequent to the last blood sampling animals were sacrificed by induction of a deep anesthesia with isoflurane followed by rapid exsanguinations. Then both nostrils and nasal cavity including pharynx were collected from all animals and preserved in neutral buffered formaldehyde (4%) for histopathological examination. Afterwards, tissues for the histopathological investigation were trimmed and embedded in paraplast^{®}. The slides were HE (hematoxylin and eosin)-stained and histopathologically examined. Histopathological evaluation showed no treatment-related effect of test item or vehicle in the nostril, nasal cavity (investigated at 4 levels) or pharynx, demonstrating very good local tolerability of Ivermectin microsuspension 5% and Ivermectin microsuspension vehicle. This not only shows that the formulation as a whole is well tolerated but also the active ingredient ivermectin itself at a dose as high as 12.5 mg/kg in rats. In addition, the results have proven that both micro- and nanosuspension show a strong and long-lasting mucoadhesive effect besides a systemic effect as seen by the Tₘₐₓ determined after a mean/median of 8.7/6 hours. Relative systemic bioavailability of both formulations was about 42 % compared to published data on a tablet formulation administered to rats.

## Claims

1. Aqueous composition comprising
- at least one water-soluble or water-dispersible natural or synthetic polymer in an amount from 0.1% by weight to 10% by weight, based on the total weight of the composition,
- at least one first surfactant in an amount from 0.1% by weight to 7.5% by weight, based on the total weight of the composition,
- at least one second surfactant in an amount from 0.1% by weight 7,5% by weight, based on the total weight of the composition,
- at least one active pharmaceutical ingredient selected from the group of avermectins in an amount from 2% by weight to 15% by weight, based on the total weight of the composition, and
- water in an amount from 60% by weight to 97.7% by weight, based on the total weight of the composition.

2. Aqueous composition according to claim 1, wherein the at least one polymer is present in the composition in an amount from 1% by weight to 10% by weight, preferably from 1% by weight to 5% by weight, more preferably in an amount from 2% by weight to 3% by weight, based on the total weight of the composition.

3. Aqueous composition according to claim 1 or claim 2, wherein the at least one polymer is selected from the group of polymers consisting of cellulose derivatives, in particular hydroxypropylmethylcellulose, polyvinylpyrrolidones and derivatives thereof, polyoxyethylene-polyoxypropylene copolymers, tocopherol-polyethylene glycol, and polyalkylene glycols, in particular polyethylene glycols.

4. Aqueous composition according to anyone of the preceding claims, wherein the at least one first surfactant is present in the composition in an amount from 0.1% by weight to 5% by weight, preferably from 0.1% by weight to 1% by weight, more preferably in an amount from 0.1% by weight to 0.5% by weight, based on the total weight of the composition.

5. Aqueous composition according to anyone of the preceding claims, wherein the at least one first surfactant is selected from a group of ionic surfactants, consisting of dioctyl sulfosuccinate ester (DOSS) and sodium dodecyl sulfate (SDS) and/or from a group of non-ionic surfactants, consisting of polysorbate esters, in particular polyoxyethylen(20)-sorbitan-monolaurat (Tween^{®} 20), polyoxyethylen(20)-sorbitan-monopalmitat (Tween^{®} 40), polyoxyethylen(20)-sorbitan-monostearat (Tween^{®} 60), polyoxyethylen(20)-sorbitan-tristearat (Tween^{®} 65) and polyoxyethylen(20)-sorbitan-monooleat (Tween^{®} 80).

6. Aqueous composition according to anyone of the preceding claims, wherein the at least one second surfactant is present in the composition in an amount from 0.1% by weight to 5% by weight, preferably from 0.1% by weight to 2.5% by weight, more preferably in an amount from 1% by weight to 2% by weight, based on the total weight of the composition.

7. Aqueous composition according to anyone of the preceding claims, wherein the at least one second surfactant is selected from a group of ionic surfactants, consisting of sodium taurocholate (STC), sodium glycocholate (SGC) and sodium deoxycholate (SDC) and/or from a group of non-ionic surfactants consisting of tocopherol-polyethylene glycols.

8. Aqueous composition according to anyone of the preceding claims, wherein the at least one active pharmaceutical ingredient selected from the group of avermectins is ivermectin.

9. Aqueous composition according to anyone of the preceding claims, in particular according to claim 8, wherein the at least one active pharmaceutical ingredient selected from the group of avermectins is present in the composition in an amount from 4% by weight to 15% by weight, preferably from 2% by weight to 10% by weight, more preferably in an amount from 2% by weight to 8% by weight, based on the total weight of the composition.

10. Aqueous composition according to anyone of the preceding claims, wherein the aqueous composition further comprises at least one buffer, wherein preferably the at least one buffer is present in an amount from 0.001% by weight to 10% by weight, preferably from 0.1% by weight to 8% by weight, more preferably from 0.14% by weight to 6% by weight, based on the total weight of the composition.

11. Aqueous composition according to claim 10, wherein the at least one buffer is selected from a group of inorganic salts consisting of monosodium phosphate, disodium phosphate, sodium bicarbonate, dicalcium phosphate, tris(hydroxymethyl) aminomethane (TRIS), sodium citrate and sodium acetate and/or from a group of organic compounds consisting of glycine, gluconic acid and chelating agents, in particular ethylenediaminetetraacetic acid (EDTA).

12. Aqueous composition according to any one of the preceding claims, wherein the at least one active pharmaceutical ingredient has a mean particle size of 0.01 µm to 20 µm, wherein it is further preferred that the at least one active pharmaceutical ingredient has a mean particle size in particular of less than 0.5 µm, wherein a mean particle size from 0.1 µm to 0.5 µm is even further preferred and/or the at least one active pharmaceutical ingredient has a mean particle size of less than 10 µm, in particular of between 1 µm and 5 µm, wherein a mean particle size between 1 µm to 3 µm is even further preferred.

13. Aqueous composition according to any one of the preceding claims, wherein
- the at least one water-soluble or water-dispersible natural or synthetic polymer is hydroxypropylmethylcellulose, present in an amount from 2% by weight to 3% by weight, based on the total weight of the composition,
- the at least one first surfactant is polyoxyethylen(20)-sorbitan-monooleat (Tween^{®} 80), present in an amount from 0.1% by weight to 0.5% by weight, based on the total weight of the composition,
- the at least one second surfactant is sodium glycocholate (SGC), present in an amount from 1% by weight to 2% by weight, based on the total weight of the composition,
- the at least one active pharmaceutical ingredient selected from the group of avermectins is ivermectin, present in an amount from 2% by weight to 8% by weight, based on the total weight of the composition,
- water is present in an amount from 86% by weight to 94.9% by weight, based on the total weight of the composition, and
- the at least one active pharmaceutical ingredient has a mean particle size of 0.1 µm to 0.5 µm.

14. Aqueous composition according to any one of the preceding claims, wherein
- the at least one water-soluble or water-dispersible natural or synthetic polymer is hydroxypropylmethylcellulose, present in an amount from 2% by weight to 3% by weight, based on the total weight of the composition,
- the at least one first surfactant is polyoxyethylen(20)-sorbitan-monooleat (Tween^{®} 80), present in an amount from 0.1% by weight to 0.5% by weight, based on the total weight of the composition,
- the at least one second surfactant is sodium glycocholate (SGC), present in an amount from 1% by weight to 2% by weight, based on the total weight of the composition, and
- the at least one active pharmaceutical ingredient selected from the group of avermectins is ivermectin, present in an amount from 2% by weight to 8% by weight, based on the total weight of the composition,
- the at least one buffer is sodium citrate buffer, present in an amount from 0,14% by weight to 6% by weight, based on the total weight of the composition,
- water is present in an amount from 80% by weight to 94.7% by weight, based on the total weight of the composition, and
- the at least one active pharmaceutical ingredient has a mean particle size of 1 µm to 3 µm.

15. Aqueous composition according to any one of the preceding claims, wherein said aqueous composition is provided for nasal application, in particular in the form of a nasal spray.

16. Discharging device, in particular a blow-fill-seal device or a syringe or a spray device, wherein the discharging device is filled with the aqueous composition according to any one of claims 1 to 15.

17. Aqueous composition according to any one of claims 1 to 15, for use in a pharmaceutical composition or medicament, wherein preferably said use is for the prevention or treatment of disorders or diseases via nasal administration, in particular for the prevention or treatment of viral diseases.

18. Aqueous composition according to any one of claims 1 to 15, for use according to claim 17 in the prevention or treatment of a SARS-Covid-2 infection.
